# EUROPEAN PATENT SPECIFICATION

(11) **EP 0 362 810 B1**
(45) Date of publication and mention of the grant of the patent: **05.05.1993**
(21) Application number: 89118384.0
(22) Date of filing: 04.10.1989
(51) Int. Cl.: A61K 31/49

(54) **Antimalarial compositions using quinidine, artemisinine and its derivatives**
Antimalariazusammenstellungen, gebrauchmachend von Quinidin, Artemisinin und ihren Derivaten
Compositions contre la malaria et méthodes de traitement utilisant la quinidine, artémisinine ou leurs dérivés

(30) Priority: 07.10.1988 EP 88116605
(43) Date of publication of application: 11.04.1990
(73) Proprietor: HOECHST AKTIENGESELLSCHAFT, 65926 Frankfurt am Main (DE)
(72) Inventor: Chatterjee, Deepak Kumar, Dr., Mulund (East) Bombay 400 081 (IN); Venugopalan, Bindumadhavan, Dr., P.O. Thane Maharashtra (IN); Blumbach, Jürgen, Dr., Bombay 400 006 (IN); Iyer, Subramani Natrajan, Dr., Nabur Mulund (West) Bombay 400 080 (IN)

(56) References cited:
- IN-B-16 615 4
- CHEMICAL ABSTRACTS, vol. 96, no. 21, 1982, page 27, abstract no. 173957c, Columbus, Ohio, US; D.C. WARHURST: "Cinchona alkaloids and malaria"
- DIALOG 05625766, MEDLINE 83-89/AUG., no. 85241766; W. PETERS: "The problem of drug resistance in malaria"

## Description

The present invention is concerned with an improved antimalarial composition which employs a combination with on the one hand, one of the antimalarial agents, Artemisinine, Dihydroartemisinine, Arteether, Artemether, Artesunate and on the other hand, the antimalarial agent Quinidine alone or with Mefloquine and/or their pharmaceutically acceptable salts.

The generic names used here and elsewhere herein are taken from "Tropical Diseases Research, Seventh Programme Report", Chapter 2; Malaria, UNDP/WORLD BANK/WHO, Published by WHO, 1985. The generic name of Quinidine is taken from "Acta Leidensia", 55, 21-27, 1987 (E.H.D. Smit (1987) The redescovery of Cichonaalkaloids as antimalaria drugs. Acta Leidensia, 55, 21-27).

Drug-resistant malaria is a serious clinical and public health problem. The malaria parasite Plasmodium falciparum, has developed a versatile capacity of evading the effect of a drug either by genetic or by nongenetic (adaptive) methods. It has been demonstrated that Chloroquine-resistance in malaria parasites is a stable genetically determined character (D.C. Warhurst (1985): Drug resistance, The Pharmaceutical Journal, No. 23, 689-692). The spread of Plasmodium falciparum resistant to chloroquine and other antimalarial drugs is a major challenge to the health care programme in tropical **and** subtropical countries (Suphat Noeypatimanond, et al., (1983): Treatment of Plasmodium falciparum malaria with a combination of Amodiaquine and Tetracycline in central Thailand, Trans. R. Soc. Trop. Med. and Hyg. 73 (3), 338-340).

The present combination of antimalarial agents as defined above, and more specifically herein below, permits desirable antimalarial therapy while preventing or delaying the development of resistance.

In an animal study, Peters (W. Peters et al. (1977): The Chemotherapy of rodent malaria XXVII. Studies on Mefloquine (WR 142490). Ann. Trop. Med. and Parasit., 71, 407-418) reported that resistance can be slowed down if an antimalarial compound is administered in combination with certain other antimalarial drugs. Peters (LW. Peters (1974): Prevention of drug resistance in rodent malaria by the use of drug mixtures. Bull. W.H.O., 51, 379-383; W. Peters (1984): Drug combination, Handbook of Experimental Pharmacology, Vol. 68/11, Antimalarial drugs (ed. W. Peters and W.H.G. Richards, P.P. Berlin, Heidelberg and New York, Springer-Verlag)) also emphasised the use of rational drug combinations for the treatment, which provide a better therapeutic value. For instance, it has been shown that a triple combination of Mefloquine, Sulfadoxin, and Pyrimethamine delayed resistance development in Plasmodium berghei (B. Merkli, et al., (1980): The inhibitory effect of a drug combination on the development of Mefloquine resistance in Plasmodium berghei. Ann. Trop. Med. and Parasit., 4(1) : 1 - 9).

The use of combinations of different antimalarials is known in malarial chemotherapy. For example, a combination of Amodiaquine and Tetracycline and a combination of Pyrimethamine and Sulphadoxine known as Fansidar have been used in the clinic [Suphat Noeypatimanond, et al., (1983): Treatment of Plasmodium falciparum malaria with a combination of Amodiaquine and Tetracycline in central Thailand, Trans. R. Soc. Trop. Med. and Hyg. 73 (3), 338 - 340]. Recently, one more antimalarial combination (Fansimef, Mefloquine, Pyrimethamine and Sulphadoxine) is undergoing clinical trials [Tropical Diseases Research, Seventh Programme Report "Chapter 2; Malaria, UNDP/WORLD BANK/WHO, Published by WHO, 1985].

Also the synergistic effect of a combination of Artemisinine and Primaquine is known (Wan Yaode, Cang Qizhong, Pharmacy Bulletin, Vol. 16, No. 1, 1981).

There are no prior reports to our knowledge concerning the clinical use of a combination of Artemisinine, Dihydroartemisinine, Arteether, Artemether, or Artesunate with Quinidine and pharmaceutically acceptable salts thereof.

The present invention concerns a new and improved antimalarial composition which comprises one or more of the compounds Artemisinine, Dihydroartemisinine, Arteether, Artemether, Artesunate in amounts less than the recommended therapeutic dose in combination with Quinidine alone or with Mefloquine or pharmaceutically acceptable salts thereof, also in sub-curative doses.

The present invention can be used for an improved method for the treatment of malaria in a mammal, including man, which comprises, in addition to treatment with the antimalarial agent Quinidine alone or with Mefloquine or pharmaceutically acceptable salts thereof in amounts less than the recommended therapeutic dose, treatment with subcurative doses of Artemisinine, Dihydroartemisine, Arteether, Artemether and/or Artesunate.

Synergistic acitivity against Plasmodium berghei is also found in murine model when either Artemisine or Dihydroartemisinine or Arteether or Artemether or Artesunate is used in combination with Mefloquine and Quinidine. This triple combination shows extraordinary synergism against rodent malaria.

The antimalarial agents, Artemisinine, Dihydroartemisinine, Arteether, Artemether, Artesunate of the first group of the present invention are known. Artemisinine has been isolated from Artemisia annua L. and subsequently synthesised. It has been used for the treatment of falciparum malaria [H.P. Koch (1981): Qinghasosu: a potent antimalarial from plant origin, Pharmacy International (New Drugs), p. 184-185, Elsevier North Holland Biomedical Press;L.J. Bruce-Chwatt (1982): Qinghaosu: a new antimalarial. British Med. J., 184, 767-768]. The clinical evaluation of the activity of Artemisinine in 2069 patients was reported by Koch in 1981, of which 1511 patients were treated for a vivax malaria [H.P. Koch (1981): Qinghaosu: a potent antimalarial from plant origin, Pharmacy International (New Drugs), p. 184-185, Elsevier North Holland Biomedical Press]. It has also been shown to be active against Chloroquine-resistant strains of Plasmodium falciparum in man[J.B. Jiang et al., (1982): Antimalarial activity of Mefloquine and Qinghaosu. Lancet, ii. 8293, 285-287]. Dihydroartemisinine, Arteether, Artemether, Artesunate are semi-synthetic derivatives of Artemisinine. Their antimalarial activity is disclosed in different WHO reports. [W.H.O. Report of the Scientific Working Group on the Chemotherapy Malaria, TDR/Chemal 3rd Review, 85. 3, Geneva, 3 - 5 June 1985 and references contained therein].

The antimalarial agents, Quinidine and Mefloquine are also known in the literature [W.H. Wernsdorfer (1987): Quinidine in health care in the tropics. Acta Leidensia, 55: 197 - 208, W. Peters (1987): Chemotherapy and drug resistance in malaria, Vol: 1, pp 240-242, Vol. 2, pp 790-91, Academic Press Ltd. London].

The clinical value of the present improved formulation in antimalarial therapy is reflected by appropriate animal studies. Typical experimental protocols, in which the ability of the test compound to act as an antimalarial agent was determined even against drug-resistant strains of P. berghei, are found in the specific examples given below.

The present invention is readily carried out. Artemisinine or one of its derivatives as defined above is generally dosed in a mammal in the range of 0.125 to 10 mg/kg x 5 days, each day one single dose. The second antimalarial agent Quinidine alone or with Mefloquine can be dosed separately, in which case the latter will be employed in an amount within (but generally lower) the dosage range and according to regimens (frequency, routes and compositions) as specified for its utility in the prior art, for example, in the references cited above or further cited in the said references.

Preferably and conveniently, Artemisinine or one of its derivatives and the second antimalarial agent of the invention are administered in a single, combined formulation. This can be in a form suitable for parenteral administration, but is preferably in a form suitable for oral administration. The proportion of each drug in the proposed combined dosage form will be in the ratio of the total daily dose of each drug when dosed alone. The combined drugs will be dosed in single or divided doses.

In the preferred oral route of dosage, the amount of Artemisinine for an average adult patient may generally be in the range of 0.2 - 2 g in combination with 200 - 600 mg Quinidine. The amount administered in the second dose may be administered 6 hours after in the range of 0.2 - 2 g of Artemisinine in combination with 100 - 300 mg of Quinidine for 3 more days, each day one single dose.

In a similar manner, combinations can be prescribed for Quinidine with other antimalarial Artemisinine derived agents of the first group. A combination of Dihydroartemisinine (range 0.2 - 1.5 g) with Quinidine (range 200 - 600 mg) may generally be administered to an adult patient followed by a 2nd dose after 6 hours in the range of 0.2 - 1.5 g of Dihydroartemisinine in combination with 200 - 600 mg of Quinidine. The amount administered in the second dose may generally be given for 3 more days, each day single dose.

Again a combination of Arteether (range 0.2 - 1.5 g) together with Quinidine as the first dose may generally be administered to an adult patient. Second dose may be administered 6 hours after the first dose and may contain 0.2 - 1.5g of Arteether plus 200 - 600 mg of Quinidine. The amount administered in the second dose may generally be given for 3 more days, each day single dose. The above mentioned Quinidine doses can also be substituted by the same dose of a mixture of Quinidine and Mefloquine.

The combined compounds are administered alone or in further combination with pharmaceutically acceptable carriers or diluents both orally and parenterally. For oral use, suitable pharmaceutical carriers include inert diluents or fillers, thereby forming dosage forms such as tablets, powders, capsules, and the like. These pharmaceutical compositions can, if desired, contain additional ingredients such as flavourings, binders, excipients and the like.

For example, tablets containing various disintegrants such as starch, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid compositions of a similar type may also be employed as fillers in soft and hard gelatin capsules, preferred materials therefore include lactose or milk sugar and high molecular weight polyethylene glycols.

For oral formulation the mixture of the compounds can for example be administered in a gelatin capsule. Such formulation could be based on a suitable refined edible oil such as sunflower oil, corn oil, peanut oil, coconut oil or til oil.

The present invention is illustrated by the following examples. It should be, however, understood that the invention is not limited to the specific details of the examples.

### Example 1

Synergistic or additive therapeutic effects of sub-curative doses of Artemisinine in combination with sub-curative doses of Quinidine against Chlorquine sensistive Plasmodium berghei infection in Swiss mice.

### Biological Evaluation Methodology:

The evaluation of blood-schizontocidal activity "28-day test" described by Raether and Fink [W. Raether and E. Fink (1979). Antimalarial activity of Floxacrine (HOE 991), I Studies on blood schizontocidal action of Floxacrine against P. berghei, P. vinekei and P. cynomolgi, Ann. Trop. Med. and Agrasit 73: 503 - 526] was followed.

**Mice:** All experiments were carried out in random bred male and female Swiss mice obtained from the Hoechst breeding house at Mulund, Bombay. The animals were free from Eperythrozoon coccoides. The animals received food pellets and water ad lib and were kept at 22 - 25°C room temperature.

**Parasite:** Plasmodium berghei K-173 strain drug-sensitive and P. berghei (NS) moderately resistant to Chloroquine were obtained from London School of Hygiene and Tropical Medicines. The strains produce lethal infection at 1 x 10⁷ parasitized red blood cells per mouse when inoculated intraperitoneally.

**Administration of compounds:** The compounds were administered orally or sub-cutaneously as per methods described by Raether and Fink [W.Raether and E. Fink (1979): Antimalarial activity of Floxacrine (Hoe 991), I, Studies on blood schizontocidal action of Floxacrine against P. berghei, P. vinekei and P. cynomolgi, Ann. Trop. Ned. and Agrasit 73: 503 - 526], Artemisinine, Dihydroartemisinine and Arteether were homogenized in double refined corn oil and such suspensions were used for sub-cutaneous inoculation in mice. Drugs were administered for 5 days. 1st dosing was done within 2 hours of infection (D+0) followed by D+1, D+2, D+3 and D+4.

**Observation on the treated mice:** The blood smears were prepared at different intervals from D+4 and continued up to D+28. Blood smears were drawn from the terminal end of the tail and stained in Giemsa. Mice which were free from P. berghei on D+28 were considered as completely cured. At least 12 mice were tested for each dosage.

The synergistic or additive therapeutic effect of sub-curative doses of Artemisinine, Dihydroartemisinine, and Arteether, each in combination with sub-curative doses of Quinidine against Chloroquine-sensitive Plasmodium berghei infected mice is shown in Table 1 when the compounds are orally administered, and in Table II when the compounds are administered sub-cutaneously. These data show that sub-curative doses of Artemisinine, Dihydroartemisinine, and Arteether, each in combination with sub-curative doses of Quinidine cure Chloroquine-sensitive Plasmodium berghei infected mice, when the compounds are administered either orally or sub-cutaneously.

### Example 2

Synergistic or additive therapeutic effect of sub-curative doses of Artemisinine in combination with sub-curative doses of Quinidine against Chloroquine-resistant strains of Plasmodium berghei (NS) infected in Swiss mice.

The evaluation of blood-schizontocidal activities was carried out following the procedure described in Example 1, using the Chloroquine resistant strain of P. berghei.

The synergistic or additive therapeutic effect of sub-curative doses of Artemisinine, Dihydroartemisinine, Arteether each in combination with sub-curative doses of Quinidine against Chloroquine-resistant strains of Plasmodium berghei (NS) infection in Swiss mice is shown in Table II when the compounds are administered either orally or sub-cutaneously. These data show that sub-curative doses of Artemisinine, Dihydroartemisinine, Arteether each in combination with sub-curative doses of Quinidine completely cured against Chloroquine-resistant strains of Plasmodium berghei (NS) infected mice, when the compounds are administered orally or sub-cutaneously.

### Example 3

Synergistic or additive potentiation effects of sub-curative doses of Artemisinine or Dihydroartemisinine or Arteether each in combination with sub-curative doses of Mefloquine plus Quinidine against chloroquine-sensitive Plasmodium Berghei K-173 infection in Swiss mice is shwon in Table III, when the compounds are administered either orally or sub-cutaneously. These data show that sub-curative doses of Artemisinine, Dihydroartemisine, Arteether each in combination with sub-curative doses of Mefloquine plus Quinidine completely cure malaria infection in mice when the compounds are administered either orally or subcutaneously. These triple combinations are novel and more effective than each drug when they are used alone.

The combination mentioned in the earlier paragraph when tested against Chloroquine-resistant strain of Plasmodium berghei NS also showed high potentiation of activity under similar routes of administration.

**Table I**

| Combination of Artemisinine or Dihydroartemisinine or Arteether with Quinidine against P. berghei K-173 | | | | |
|---|---|---|---|---|
| Formulations | Route | Dose mg/kg x 5 | No. of mice per group | infected animals (% cured) |
| Artemisinine (curative dose) | p.o. | 200 | 12 | 100 |
| | s.c. | 20 | 12 | 100 |
| Artemisinine (sub-curative dose) | p.o. | 100 | 20 | 40 |
| | s.c. | 1.0 | 12 | 25 |
| Dihydroartemisinine (curative dose) | p.o. | 100 | 12 | 100 |
| | s.c. | 4 | 12 | 100 |
| Dihydroartemisinine (sub-curative dose) | p.o. | 50 | 12 | 50 |
| | s.c. | 2.5 | 12 | 50 |
| Arteether (curative dose) | p.o. | 50 | 12 | 100 |
| | s.c. | 7.5 | 16 | 100 |
| Arteether (sub-curative dose) | p.o. | 10 | 20 | 0 |
| | s.c. | 5 | 16 | 68 |
| Quinidine (curative dose) | p.o. | 200 | 12 | 100 |
| | s.c. | 50 | 12 | 100 |
| Quinidine (sub-curative dose) | p.o. | 100 | 12 | 0 |
| | s.c. | 25 | 12 | 40 |
| Artemisinine + Quinidine | p.o. | 25 + 100 | 12 | 83 |
| | s.c. | 2.5+ 50 | 12 | 83 |
| Dihydroartemisinine + Quinidine | p.o. | 50 + 100 | 12 | 100 |
| | s.c. | 1.25+50 | 12 | 100 |
| Arteether + Quinidine | p.o. | 10 - 75 | 20 | 100 |
| | s.c. | 2.5+ 50 | 20 | 100 |

**Table II**

| Combination of Artemisinine or Dihydroartemisinine or Arteether with Quinidine against P. berghei NS strain (moderately resistant to Chloroquine) | | | | |
|---|---|---|---|---|
| Formulations | Route | Dose mg/kg x 5 | No. of mice per group | infected animals (% cured) |
| Artemisinine (sub-curative dose) | p.o. | 200 | 12 | 50 |
| | s.c. | 20 | 12 | 50 |
| Dihydroartemisinine (sub-curative dose) | p.o. | 100 | 12 | 50 |
| | s.c. | 5 | 12 | 50 |
| Arteether (sub-curative dose) | p.o. | 15 | 12 | 25 |
| | s.c. | 5 | 12 | 33 |
| Quinidine (sub-curative dose) | p.o. | 100 | 12 | 0 |
| | s.c. | 25 | 12 | 33 |
| Artemisinine + Quinidine | p.o. | 100 + 100 | 12 | 83 |
| | s.c. | 10 + 20 | 12 | 75 |
| Dihydroartemisinine + Quinidine | p.o. | 50 + 100 | 12 | 100 |
| | s.c. | 5 + 20 | 12 | 100 |
| Arteether + Quinidine | p.o. | 10 + 100 | 12 | 100 |
| | s.c. | 2.5 + 20 | 12 | 83 |

**Table III**

| Combination of Artemisinine or Dihydroartemisinine or Arteether with Quinidine against P. berghei K-173 | | | | |
|---|---|---|---|---|
| Formulations | Route | Dose mg/kg x 5 | No. of mice per group | infected animals (% cured) |
| Artemisinine (curative dose) | p.o. | 200 | 12 | 100 |
| | s.c. | 20 | 12 | 100 |
| Artemisinine (sub-curative dose) | p.o. | 100 | 20 | 40 |
| | s.c. | 1.0 | 12 | 25 |
| Dihydroartemisinine (curative dose) | p.o. | 100 | 12 | 100 |
| | s.c. | 4 | 12 | 100 |
| Dihydroartemisinine (sub-curative dose) | p.o. | 50 | 12 | 50 |
| | s.c. | 2.5 | 12 | 50 |
| Arteether (curative dose) | p.o. | 50 | 12 | 100 |
| | s.c. | 7.5 | 16 | 100 |
| Arteether (sub-curative dose) | p.o. | 10 | 20 | 0 |
| | s.c. | 5 | 16 | 68 |
| Quinidine (curative dose) | p.o. | 200 | 12 | 100 |
| | s.c. | 50 | 12 | 100 |
| Quinidine (sub-curative dose) | p.o. | 100 | 12 | 0 |
| | s.c. | 25 | 12 | 40 |
| Mefloquine (curative dose) | p.o. | 7 | 12 | 100 |
| | s.c. | 5 | 12 | 100 |
| Mefloquine (sub-curative dose) | p.o. | 4 | 12 | 50 |
| | s.c. | 2.5 | 12 | 25 |
| Arteether + Mefloquine + Quinidine | p.o. | 7.5+2.5+75 | 16 | 100 |
| | s.c. | 2.5+2.5+25 | 12 | 100 |
| Artemisinine + Mefloquine + Quinidine | p.o. | 25+2.5+75 | 8 | 100 |
| | s.c. | 2.5+2.5+25 | 8 | 100 |
| Dihydroartemisinine + Mefloquine + Quinidine | p.o. | 40+2.5+50 | 12 | 100 |
| | s.c. | 2+2.5+25 | 12 | 100 |

## Claims (Claims for the following Contracting State(s): AT, BE, CH, DE, FR, GB, IT, LI, NL)

1. A pharmaceutical combination with a synergistic action against malaria, which optionally besides customary auxiliaries and vehicles, contains one or more compounds selected from the group Artemisinine, Dihydroartemisinine, Arteether, Artemether and Artesunate, as well as the pharmacologically tolerated salts thereof (group 1), and Quinidine alone or with Mefloquine or their pharmaceutically tolerated salts.

2. A pharmaceutical combination as claimed in claim 1, which contains one or more compounds from the group comprising Artemisinine, Dihydroartemisinine, Arteether, Artemether, Artesunate (group 1) and Quinidine alone or with Mefloquine.

3. A pharmaceutical combination as claimed in claim 1, which contains one compound from group (1) and Quinidine.

4. A pharmaceutical combination as claimed in claim 1, which contains Artemisinine and Quinidine.

5. A pharmaceutical combination as claimed in claim 1, which contains Dihydroartemisinine and Quinidine.

6. A pharmaceutical combination as claimed in claim 1, which contains Arteether and Quinidine.

7. A pharmaceutical combination as claimed in claim 1, which contains Artemether and Quinidine.

8. A pharmaceutical combination as claimed in claim 1, which contains Artesunate and Quinidine.

9. A pharmaceutical combination as claimed in claim 1, which contains Arteether or Artemisinine or Dihydroartemisinine or Artemether or Artesunate and Mefloquine and Quinidine.

10. The use of a combination of compounds as claimed in claims 1 - 9 for the preparation of pharmaceuticals with a synergistic and/or potentiating activity against malaria.

## Claims (Claims for the following Contracting State(s): ES)

1. A process for the production of a pharmaceutical combination with a synergistic action against malaria, wherein one or more compounds selected from the group Artemisinine, Dihydroartemisinine, Arteether, Artemether and Artesunate, as well as the pharmacologically tolerated salts thereof (group 1), and Quinidine alone or with Mefloquine or their pharmaceutically tolerated salts are optionally together with customary auxiliaries and vehicles, transformed into a form suitable for administration.

2. A process as claimed in claim 1, wherein the compounds of group (1) are Artemisinine, Dihydroartemisinine, Arteether, Artemether, Artesunate.

3. A process as claimed in claim 1, wherein one compound of group (1) is combined with Quinidine.

4. A process as claimed in claim 1, wherein Artemisinine is combined with Quinidine.

5. A process as claimed in claim 1, wherein Dihydroartemisinine is combined with Quinidine.

6. A process as claimed in claim 1, wherein Arteether is combined with Quinidine.

7. A process as claimed in claim 1, wherein Artemether is combined with Quinidine.

8. A process as claimed in claim 1, wherein Artesunate is combined with Quinidine.

9. A process as claimed in claim 1, wherein Arteether or Artemisinine or Dihydroartemisinine or Artemether or Artesunate is combined with Mefloquine and Quinidine.

10. The use of a combination of compounds as claimed in claims 1 - 9 for the preparation of pharmaceuticals with a synergistic and/or potentiating activity against malaria.

## Patentansprüche (Patentansprüche für folgende(n) Vertragsstaat(en): AT, BE, CH, DE, FR, GB, IT, LI, NL)

1. Pharmazeutische Kombination mit synergistischer Wirkung gegenüber Malaria, die, neben fakultativen üblichen Hilfsstoffen und Trägern, eine oder mehrere, aus der Gruppe Artemisinin, Dihydroartemisinin, Arteether, Artemether und Artesunat sowie den pharmakologisch verträglichen Salzen hievon ausgewählte Verbindungen (Gruppe 1) und Chinidin allein oder zusammen mit Mefloquin oder ihren pharmazeutisch verträglichen Salzen enthält.

2. Pharmazeutische Kombination nach Anspruch 1, die eine oder mehrere Verbindungen aus der Artemisinin, Dihydroartemisinin, Arteether, Artemether und Artesunat (Gruppe 1) umfassenden Gruppe und Chinidin allein oder zusammen mit Mefloquin enthält.

3. Pharmazeutische Kombination nach Anspruch 1, die eine Verbindung aus Gruppe (1) und Chinidin enthält.

4. Pharmazeutische Kombination nach Anspruch 1, die Artemisinin und Chinidin enthält.

5. Pharmazeutische Kombination nach Anspruch 1, die Dihydroartemisinin und Chinidin enthält.

6. Pharmazeutische Kombination nach Anspruch 1, die Arteether und Chinidin enthält.

7. Pharmazeutische Kombination nach Anspruch 1, die Artemether und Chinidin enthält.

8. Pharmazeutische Kombination nach Anspruch 1, die Artesunat und Chinidin enthält.

9. Pharmazeutische Kombination nach Anspruch 1, die Arteether oder Artemisinin oder Dihydroartemisinin oder Aremether oder Artesunat und Mefloquin und Chinidin enthält.

10. Verwendung einer Kombination von Verbindungen nach den Ansprüchen 1 bis 9 zur Herstellung von Arzneimitteln mit synergistischer und/oder potenzierender Aktivität gegenüber Malaria.

## Patentansprüche (Patentansprüche für folgende(n) Vertragsstaat(en): ES)

1. Verfahren zur Herstellung einer pharmazeutischen Kombination mit synergistischer Wirkung gegenüber Malaria, worin eine oder mehrere Verbindungen, ausgewählt aus der Gruppe Artemisinin, Dihydroartemisinin, Arteether, Artemether und Artesunat sowie den pharmakologisch verträglichen Salzen hievon (Gruppe 1), und Chinidin allein oder zusammen mit Mefloquin, oder ihre pharmazeutisch verträglichen Salze, gegebenenfalls zusammen mit üblichen Hilfsstoffen und Trägern, in eine für die Verabreichung geeignete Form gebracht werden.

2. Verfahren nach Anspruch 1, worin die Verbindungen der Gruppe (1) Artemisinin, Dihydroartemisinin, Arteether, Artemether, Artesunat sind.

3. Verfahren nach Anspruch 1, worin eine Verbindung der Gruppe (1) mit Chinidin kombiniert wird.

4. Verfahren nach Anspruch 1, worin Artemisinin mit Chinidin kombiniert wird.

5. Verfahren nach Anspruch 1, worin Dihydroartemisinin mit Chinidin kombiniert wird.

6. Verfahren nach Anspruch 1, worin Arteether mit Chinidin kombiniert wird.

7. Verfahren nach Anspruch 1, worin Artemether mit Chinidin kombiniert wird.

8. Verfahren nach Anspruch 1, worin Artesunat mit Chinidin kombiniert wird.

9. Verfahren nach Anspruch 1, worin Arteether oder Artemisinin oder Dihydroartemisinin oder Artemether oder Artesunat mit Melfloquin und Chinidin kombiniert wird.

10. Verwendung einer Kombination von Verbindungen nach den Ansprüchen 1 bis 9 zur Herstellung von Arzneimitteln mit synergistischer und/oder potenzierender Aktivität gegenüber Malaria.

## Revendications (Revendications pour l'(les) Etat(s) contractant(s) suivant(s): AT, BE, CH, DE, FR, GB, IT, LI, NL)

1. Association pharmaceutique ayant une action synergique contre le paludisme, qui, éventuellement en plus d'auxiliaires et véhicules usuels, contient un ou plusieurs composés choisis parmi l'artemisinine, la dihydroartémisinine, l'artééther, l'artéméther et l'artésunate, ainsi que leurs sels pharmacologiquement acceptables (groupe 1), et de la quinidine seule ou avec de la méfloquine ou leurs sels pharmaceutiquement acceptables.

2. Association pharmaceutique selon la revendication 1, contenant un ou plusieurs composés choisis parmi l'artémisinine, la dihydroartémisinine, l'artééther, l'artéméther, l'artésunate (groupe 1) et de la quinidine seule ou avec de la méfloquine.

3. Association pharmaceutique selon la revendication 1, contenant un composé du groupe (1) et de la quinidine.

4. Association pharmaceutique selon la revendication 1, contenant de l'artémisinine et de la quinidine.

5. Association pharmaceutique selon la revendication 1, contenant de la dihydroartémisinine et de la quinidine.

6. Association pharmaceutique selon la revendication 1, contenant de l'artééther et de la quinidine.

7. Association pharmaceutique selon la revendication 1, contenant de l'artéméther et de la quinidine.

8. Association pharmaceutique selon la revendication 1, contenant de l'artésunate et de la quinidine.

9. Association pharmaceutique selon la revendication 1, contenant de l'artééther ou de l'artémisinine ou de la dihydroartémisinine ou de l'artéméther ou de l'artésunate et de la méfloquine et de la quinidine.

10. Utilisation d'une association de composés selon les revendications 1 à 9, pour la préparation de produits pharmaceutiques ayant une activité synergique et/ou potentialisante contre le paludisme.

## Revendications (Revendications pour l'(les) Etat(s) contractant(s) suivant(s): ES)

1. Procédé pour la préparation d'une association pharmaceutique ayant une action synergique contre le paludisme, dans lequel on met sous une forme convenable pour l'administration un ou plusieurs composés choisis parmi l'artemisinine, la dihydroartémisinine, l'artééther, l'artéméther et l'artésunate, ainsi que leurs sels pharmacologiquement acceptables (groupe 1), et de la quinidine seule ou avec de la méfloquine ou leurs sels pharmaceutiquement acceptables, éventuellement conjointement avec des auxiliaires et véhicules usuels.

2. Procédé selon la revendication 1, dans lequel les composés du groupe (1) sont l'artémisinine, la dihydroartémisinine, l'artééther, l'artéméther, l'artésunate.

3. Procédé selon la revendication 1, dans lequel un composé du groupe (1) est associé à la quinidine.

4. Procédé selon la revendication 1, dans lequel l'artémisinine est associée à la quinidine.

5. Procédé selon la revendication 1, dans lequel la dihydroartémisinine est associée à la quinidine.

6. Procédé selon la revendication 1, dans lequel l'artééther est associé à la quinidine.

7. Procédé selon la revendication 1, dans lequel l'artéméther est associé à la quinidine.

8. Procédé selon la revendication 1, dans lequel l'artésunate est associé à la quinidine.

9. Procédé selon la revendication 1, dans lequel l'artééther ou l'artémisinine ou la dihydroartémisinine ou l'artéméther ou l'artésunate est associé à la méfloquine et la quinidine.

10. Utilisation d'une association de composés selon les revendications 1 à 9, pour la préparation de produits pharmaceutiques ayant une activité synergique et/ou potentialisante contre le paludisme.
